# EUROPÄISCHE PATENTSCHRIFT

(11) **EP 4 041 130 B1**
(45) Veröffentlichungstag und Bekanntmachung des Hinweises auf die Patenterteilung: **13.05.2026**
(21) Anmeldenummer: 20816100.0
(22) Anmeldetag: 10.11.2020
(51) Int. Cl.: A61F 2/16

(54) **INTRAOKULARLINSE MIT SOLL-BIEGESTELLE AN DER HAPTIK**
INTRAOCULAR LENS HAVING A TARGET BENDING POINT ON THE HAPTIC
LENTILLE INTRAOCULAIRE AYANT UN POINT DE COURBURE CIBLE SUR L'HAPTIQUE

(30) Priorität: 15.11.2019 DE 102019130904
(43) Veröffentlichungstag der Anmeldung: 17.08.2022
(73) Patentinhaber: SUTTER, Florian, 9100 Herisau (CH)
(72) Erfinder: SUTTER, Florian, 9100 Herisau (CH)
(74) Vertreter: RavensPAT Patentanwälte Partnerschaft mbB
(86) Internationale Anmeldenummer: PCT/EP2020/081628
(87) Internationale Veröffentlichungsnummer: WO 2021/094306

(56) Entgegenhaltungen:
- EP-A1- 1 591 080
- FR-A1- 2 918 558
- US-A1- 2019 183 632

## Beschreibung

Die Erfindung betrifft eine Intraokularlinse als Implantat zum Ersatz einer natürlichen Linse im Kapselsack eines Auges nach dem Oberbegriff des Anspruchs 1.

Aus dem Stand der Technik sind verschiedene Bauformen von Intraokularlinsen bekannt, zum Beispiel aus der CN 207136934 U oder der US 2019/183632 A1.

Aufgabe der Erfindung ist es, eine Intraokularlinse bereitstellen zu können, die beim Einsetzen ein besseres und sichereres Positionieren der optischen Linse ermöglicht.

Die Aufgabe wird, ausgehend von einer Intraokularlinse der eingangs genannten Art, durch die kennzeichnenden Merkmale des Anspruchs 1 gelöst.

Durch die in den abhängigen Ansprüchen genannten Maßnahmen sind vorteilhafte Ausführungen und Weiterbildungen der Erfindung möglich.

Typischerweise kann die erfindungsgemäße Intraokularlinse bei Erkrankungen, Altersbegleiterscheinungen oder dergleichen eingesetzt werden, bei denen die natürliche Linse im Auge, genauer gesagt, im Kapselsack ersetzt werden muss, weil diese zum Beispiel infolge des Grauen Stars (Katarakts) eingetrübt ist. Durch einen Zugang über die Hornhaut oder die Lederhaut kann die eingetrübte Linse aus dem Kapselsack entfernt werden. Der Kapselsack selbst ist an den Zonulafasern befestigt, die wiederum am Ziliarmuskel hängen. Die Intraokularlinse wird, wenn immer möglich, in den Kapselsack eingebracht. Die erfindungsgemäße Intraokularlinse hält und zentriert sich im Inneren des Kapselsackes. Sie umfasst zunächst als optischen Teil einen Linsenkörper zur Ausbildung der zentralen optischen Linse. Entsprechend weist die optische Linse eine optische Achse auf, also eine Symmetrieachse der Linse, die selbst wiederum ein im Wesentlichen rotationssymmetrisches,torisches oder multifokales optisches System darstellt.

Am Rand des Linsenkörpers ist bei der erfindungsgemäßen Intraokularlinse eine sogenannte Haptik bzw. Haptikvorrichtung angebracht, mit der die Linse im Kapselsack fixiert wird. Sie ist so ausgebildet, dass sie den Kapselsack leicht streckt und die Linse im Kapselsack wiederum zentriert, sodass ein Verrutschen der Linse im Nachhinein erschwert und die gewünschte optische Abbildung ermöglicht wird. Die Haptikvorrichtung liegt in einer Lagerungsebene senkrecht zur optischen Achse des Linsenkörpers.

Die Vorrichtung weist im vorliegenden Fall wenigstens ein Halterungselement auf, das zumindest abschnittsweise bogenförmig ausgebildet ist. Es kann sich dabei um einen zum Teil radial von der Linse abstehenden Arm handeln, bzw. um einen solchen, der leicht gekrümmt nach außen steht. Denkbar ist auch, dass geschlossene geometrische Formen wie Ringe oder dergleichen als Halterungselemente verwendet werden. Dieser Arm bzw. dieses Halterungselement ist grundsätzlich flexibel und besitzt zudem eine gewisse Elastizität, d.h. die gesamte Intraokularlinse (Haptik mit Linsenkörper) kann zum einen durch ihre Flexibilität gut auch durch schmale Zugänge ins Auge eingesetzt werden, indem die Linse zum Beispiel zusammengerollt wird. Die Linse kann sich aber auch gut an die geometrischen Gegebenheiten innerhalb des Kapselsacks anpassen. Durch eine gewisse Elastizität wiederum drückt die Haptikvorrichtung gegen den Kapselsack, spannt diesen seitlich in der Lagerungsebene und streckt den Kapselsack dabei auch leicht. Durch diese Krafteinwirkung zwischen dem Rand des Kapselsacks und der Haptikvorrichtung wird aber auch gleichzeitig der Linsenkörper aufgespannt und an einer festen Position im Kapselsack gehalten und kann grundsätzlich auch nicht mehr verrutschen. Dadurch wird die Optik optimal zentriert.

Die erfindungsgemäße Intraokularlinse zeichnet sich nunmehr dadurch aus, dass das Halterungselement wenigstens eine Soll-Biegestelle aufweist, deren Breite in der Lagerungsebene geringer ist als die Dicke in Richtung der optischen Achse. Damit über das Halterungselement eine Kraft auf den Linsenkörper ausgeübt wird, sodass dieser fixiert und zentriert wird, ist es vorteilhaft, dass der gesamte Durchmesser der Intraokularlinse einschließlich der Haptikvorrichtung im nicht implantierten Fall etwas größer ist als der Durchmesser des Kapselsacks nach Entfernung der natürlichen Linse, damit die Haptikvorrichtung bzw. die Halterungselemente leicht in radialer Richtung bzw. zur optischen Achse hin zusammengedrückt werden und durch deren Elastizität eine Kraft auf den Kapselsack bzw. auf den Linsenkörper ausübt, die diesen in seiner Position hält.

Werden die Halterungselemente beim Einpassen in den Kapselsack in radialer Richtung nach innen zur optischen Achse hin gedrückt, so wird das Halterungselement sich grundsätzlich an der Stelle verbiegen, an welcher der geringste mechanische Widerstand ist. Bei herkömmlichen Intraokularlinsen aus dem bisherigen Stand der Technik kann beim Einsetzen in den Kapselsack die radial zum Linsenkörper wirkende Kraft die Elastizität der Halterungelemente überbeanspruchen und diese damit abknicken lassen, was dazu führt, dass ein oder beide Halterungselemente zum Beispiel in Richtung der optischen Achse / senkrecht zur Lagerungsebene nachgibt und in dieser Richtung aus der Ebene verkippt wird. Als unmittelbare Folge ist dann auch der Linsenkörper bzw. dessen optische Achse zur optischen Achse des Auges verkippt angeordnet. Dies führt dazu, dass die somit implantierte herkömmliche Linse nicht die gewünschte optische Abbildung liefert, was die Sehqualität ohne zusätzliche optische Korrektur (Brille oder Kontaklinse) verschlechtert.

In der Praxis stellt man fest, dass die Linsengröße je nach Einzelfall für unterschiedliche Patienten abweicht. Kurzsichtige Patienten haben in der Regel größere Linsen als weitsichtige. Dennoch werden die Intraokularlinsen hinsichtlich ihrer Größe meist nicht individuell für den einzelnen Patienten maßgefertigt oder für verschiedene Augengrössen abgestuft hergestellt.

Infolge der erfindungsgemäßen Soll-Biegestellen ist jedoch mechanisch vorgegeben, an welcher Stelle die Halterungselemente aufgrund ihrer Flexibilität nachgeben, und dies erfolgt innerhalb der Lagerungsebene, sodass vermieden werden kann, dass der Linsenkörper verkippt. Dadurch kann die Positionierung mit einer deutlich erhöhten Präzision vorgenommen werden.

Innerhalb des Halterungselements kann die Soll-Biegestelle wie eine Singularität in der geometrischen Gestalt der Halterungselemente ausgebildet sein. Auf wenigstens einer, insbesondere beiden Seiten der Soll-Biegestelle kann sich ein Bereich anschließen, dessen Breite gemessen in der Lagerungsebene mindestens so groß ist wie die Dicke des entsprechenden Halterungselements in Richtung der optischen Achse. Grundsätzlich ist es denkbar, dass diese Soll-Biegestelle unmittelbar dort vorgesehen ist, wo das Halterungselement an den Linsenkörper ansetzt. Aus Stabilitätsgründen und zusätzlich, um die Krümmung des Halterungselements besser und gleichmäßiger an den Rand des Kapselsacks anzupassen, kann aber auch die Soll-Biegestelle bei einer Ausführungsform der Erfindung am Halterungselement weiter nach radial außen, d.h. vom Linsenkörper weiter beabstandet positioniert sein. Um eine noch bessere Anpassung des Halterungselements an die Krümmung des Randes des Kapselsacks zu erreichen, können insbesondere auch zwei oder mehrere Soll-Biegestellen an einem Halterungselement vorgesehen sein.

Zum einen kann die Soll-Biegestelle eine flexiblere Anpassung an den Kapselsack ermöglichen; andererseits wiederum kann das Halterungselement mit einer gewissen Elastizität ausgebildet sein, sodass die Intraokularlinse über das Halterungselement eine Kraft auf den Kapselsack ausübt, damit dieser leicht gestreckt wird, sich aber trotzdem an eine mögliche Schrumpfung des Kapselsackes anpassen kann. Ferner wird der Linsenkörper selbst sodann fixiert und zentriert.

Bei einer vorteilhaften Ausgestaltung der Erfindung ist die Haptikvorrichtung derart ausgebildet, dass die Halterungselemente um den Linsenkörper herum bezüglich einer Linie in der Lagerungsebene, die senkrecht durch die optische Achse verläuft, spiegelsymmetrisch und/oder in der Lagerungsebene in gleichen Winkelabständen bezüglich der Mitte des Linsenkörpers angeordnet sind. Beide Anordnungen ermöglichen grundsätzlich eine stabile und vor allem gut zentrierte Positionierung des Linsenkörpers im Auge.

Um die Intraokularlinse möglichst günstig herstellen zu können, ist die wenigstens eine Soll-Biegestelle als Aussparung, insbesondere mit glattem Randverlauf ausgebildet. Durch diese Schwächung im Bereich der Breite des Haltelements an einer Stelle kann dieses leichter beim Einbringen in den Kapselsack nachgeben und in der Lagerungsebene abknicken. Es ist kein Gelenk oder dergleichen notwendig, sondern es wird lediglich Material ausgespart. Der glatte Rand ermöglicht wiederum, dass dennoch eine gewisse Elastizität auch an der Soll-Biegestelle vorhanden ist, während bei einer mit Kanten versehenen Kerbe eher zu erwarten ist, dass das Material seine Elastizität verliert und einknickt. Grundsätzlich kann die Soll-Biegestelle von Material des Halterungselements umgeben sein, **d.h.** sie stellt ein Loch innerhalb des Materials dar. In vorteilhafter Weise kann die Soll-Biegestelle einseitig offen ausgebildet sein, sodass auch die Richtung, in der sich das Material biegt, deutlicher vorgegeben ist, d.h. in Richtung zur Öffnung hin. Diese Maßnahme bietet eine vergleichsweise einfache und kostengünstige Fertigung.

Bei einer Ausführungsvariante der Erfindung kann die Soll-Biegestelle insbesondere auf der Innenseite des jeweiligen bogenförmigen Halterungselements angeordnet sein. Aufgrund der Biegung wird sich das Halterungselement vornehmlich auch in Richtung dieser Biegung weiter krümmen. Wird die Intraokularlinse in den Kapselsack eingebracht, wird bei einer solchen C-förmigen Haptik die Linse so gedreht, dass sie der Krümmung der Halterungselemente entgegengerichtet ist, sodass die Halterungselemente nicht entgegen ihrer Krümmung umklappen. Verläuft also die Krümmung zum Beispiel linksgekrümmt und die Halterungselemente weisen z.B. aus Sicht des Beobachters in Richtung gegen den Uhrzeigersinn, so wird die Linse beim Einbringen in den Kapselsack und dem anschließenden Positionieren rechts herum gedreht, sodass sich die Halterungselemente gut an den Rand des Kapselsacks anlegen können.

Hinsichtlich der Herstellung ist es besonders vorteilhaft, wenn Linsenkörper bzw. die Hülle des Linsenkörpers und die Haptikvorrichtung selbst einteilig bzw. aus dem gleichen Material gefertigt sind. Bei herkömmlichen Intraokularlinsen aus dem Stand der Technik, bei denen Haptikvorrichtung und Linsenkörper aus unterschiedlichen Materialien gefertigt sind, müssen Haptik und Optik erst miteinander verbunden werden, indem zum Beispiel sehr kleine Löcher in den Linsenkörper gebohrt und anschließend die Haptik in diese Löcher angebracht und verklebt wird. Zum einen hat dies zwar den Vorteil, dass grundsätzlich vergleichsweise dünne Haptikelemente mit speziell durch die Materialauswahl bedingten vorteilhaften Eigenschaften ausgewählt werden können. Gleichzeitig besteht jedoch das Problem, dass nicht nur die Herstellung sehr aufwendig ist, sondern auch leichter Fehler beim Anbringen der Haptik am Linsenkörper auftreten und sich diese gegebenenfalls mit der Zeit lösen. Ferner sind derartige Linsen meist sehr teuer.

Erfindungsgemäß kommen sogenannte C-Haptiken infrage, die gekrümmte Halterungselemente aufweisen, die wie ein Arm ausgebildet sind, d.h. ein Ende des Halterungselements ist mit dem Linsenkörper verbunden, während das andere frei ist. die Haptikvorrichtung kann in der Regel mindestens zwei, insbesondere auch drei oder vier Halterungselemente umfassen. Denkbar ist auch, dass die Halterungselemente einen geschlossen Bogen aufweisen, der eine Aussparung umschließt. Somit Aussparung beim Einfügen in den Kapselsack deformiert werden. Gerade bei C-Haptiken können die Halterungselemente in der Regel in der gleichen Richtung gekrümmt angeordnet sein, sodass die Linse beim Positionieren durch Drehung in ihre endgültige Position gebracht werden kann und die Halterungselemente in Form von Armen bzw. C-Artikel ohne sich zu verdrehen in die richtige Position gemäß ihrer Krümmung finden. Auf diese Weise kann eine stabile Halterungen Positionierung der Intraokularlinse erfolgen.

Beim Einbringen einer Intraokularlinse in den Kapselsack eines Auges wird zunächst eine erfindungsgemäße Intraokularlinse bzw. einer Ausführungsform der Erfindung verwendet. Durch eine chirurgische Öffnung in der peripheren Hornhaut oder die angrenzende Lederhaut hindurch wird die Intraokularlinse durch eine Einbringungseinrichtung, ähnlich einer Spritze in den Kapselsack bewegt. In der Regel wird die Intraokularlinse durch eine vergleichsweise dicke Kanüle hindurchgedrückt. Damit dies in definierter Weise geschieht und die Intraokularlinse nicht beschädigt wird, wird diese zuvor so stark um die Einbringungsachse herum eingerollt, dass die gegenüberliegenden Enden jeweils überlappen. Gelangt die Intraokularlinse sodann in den Kapselsack, ist sie nicht mehr der mechanischen Zwangsbedingungen unterworfen, die sie in der aufgerollten Form innerhalb der Kanüle hält und klappt auseinander. Der Operateur musste dann in der Regel die Linse noch einmal positionieren und zum Beispiel soweit drehen, bis sich die Halterungselemente hinreichend an den Rand des Kapselsachs anschmiegen, die Linse zentrieren und gleichzeitig den Kapselsack leicht nach außen spannen.

### Ausführungsbeispiele

Ausführungsbeispiele der Erfindung sind in der Zeichnung dargestellt und werden nachstehend unter Angabe weiterer Einzelheiten und Vorteile näher erläutert. Im Einzelnen zeigen:
- Figur 1:: eine Intraokularlinse gemäß der Erfindung mit einer Soll-Biegestelle im Halterungselement, sowie
- Figur 2:: eine Intraokularlinse gemäß Erfindung mit zwei Soll-Biegestellen an jeweils einem Halterungselement.

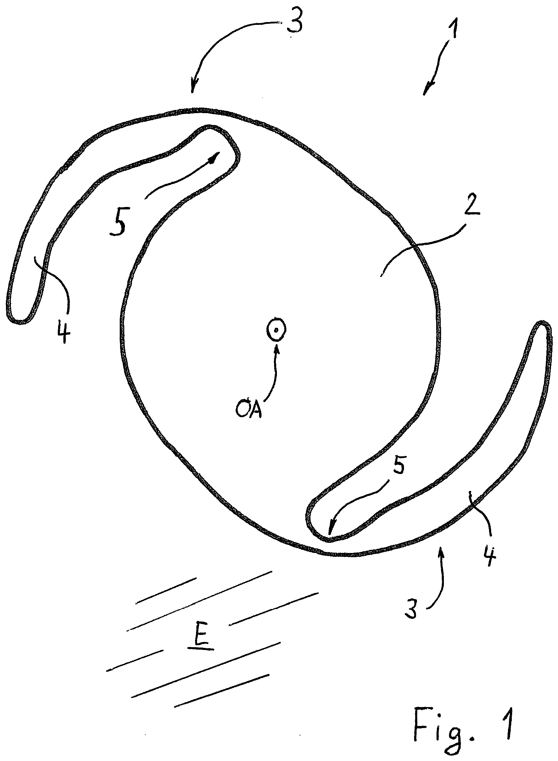

Figur 1 zeigt eine Intraokularlinse 1 mit Blick in Richtung der optischen Achse OA gemäß der Erfindung mit einem Linsenkörper 2, wobei die Haptikvorrichtung 3 zwei um 180° zueinander versetzte Halterungselemente 4 umfasst, die als C-Haptik ausgebildet sind. Folgt man einem der Halterungselemente 4 vom Linsenkörper 2 radial nach außen, also von der Stelle aus, an der das jeweilige Halterungselement 4 am Linsenkörper 2 ansetzt, so ist zu sehen, dass sich das jeweilige Halterungselement 4 sich an der Soll-Biegestelle 5 einschnürt. Die Einschnürung kommt durch eine Aussparung am inneren Rand der Krümmung des Halterungselements 4 zustande.

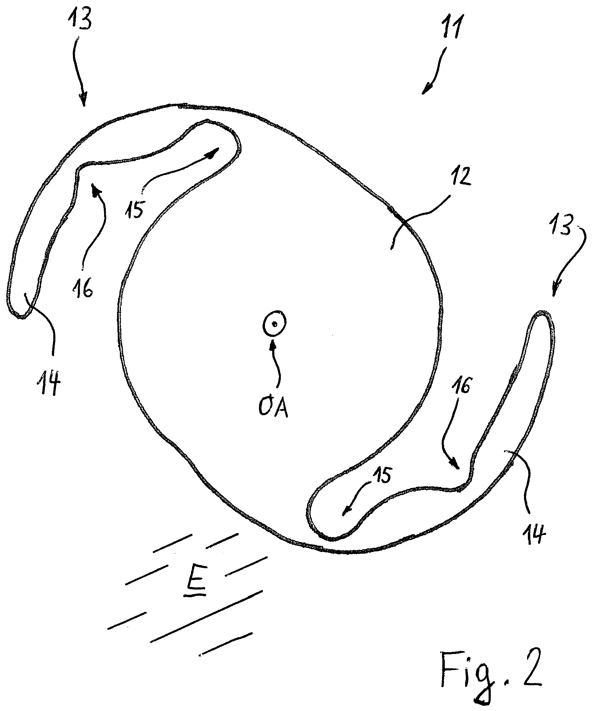

Figur 2 wiederum zeigt 1 mit Blick in Richtung der optischen Achse OA eine ähnliche Intraokularlinse 11 mit einem Linsenkörper 12 und einer Haptikvorrichtung 13, die analog zur Ausführungsform gemäß Figur 1 um 180° zueinander versetzte Halterungselemente 14 aufweist. Im Unterschied zur Ausführungsform nach Figur 1 sind jedoch pro Haltungselement 14 zwei Soll-Biegestellen 15, 16 vorgesehen. Da diese Soll-Biegestellen 15, 16 in unmittelbarer Nähe aufeinanderfolgen, geht es hier vor allem um eine hinreichende Anpassung der Flexibilität. Die Intraokularlinsen 1, 11 gemäß den Figuren 1 und 2 sind einstückig ausgebildet.

Die Zeichenebene der Figuren 1 und 2 entspricht der Lagerungsebene E (auch im Kapselsack).

### Bezugszeichen:

- 1: Intraokularlinse (IOL)
- 2: Linsenkörper
- 3: Haptikvorrichtung
- 4: Halterungselemente
- 5: Soll-Biegestelle
- 11: Intraokularlinse
- 12: Linsenkörper
- 13: Haptikvorrichtung
- 14: Halterungselemente
- 15: Soll-Biegestelle
- 16: Soll-Biegestelle
- E: Lagerungsebene
- OA: optische Achse der IOL

## Patentansprüche

1. Intraokularlinse (1, 11) als Implantat zum Ersatz einer natürlichen Linse im Kapselsack eines Auges, umfassend:
• einen Linsenkörper (2, 12) zur Ausbildung der zentralen optischen Linse, die eine optische Achse (OA) aufweist,
• eine am Rand des Linsenkörpers (2, 12) angebrachte Haptikvorrichtung (3, 13) zum Fixieren der Intraokularlinse (1, 11) im Kapselsack und/oder zum Strecken des Kapselsacks und/oder zum Zentrieren des Linsenkörpers (2, 12) im Kapselsack,
• wobei die Haptikvorrichtung (3, 13) in einer Lagerungsebene (E) liegt, die senkrecht zur optischen Achse (OA) des Linsenkörpers (2, 12) liegt,
• wobei die Haptikvorrichtung (3, 13) wenigstens ein wenigstens abschnittsweise bogenförmiges Halterungselement (4, 14) umfasst,
• wobei das wenigstens eine Halterungselement (4, 14) wenigstens eine Soll-Biegestelle (5, 15, 16) aufweist, wobei die Soll-Biegestelle oder mehrere Sollbiegestellen (15, 16) an wenigstens einem der Halterungselemente (14) als Aussparung mit glattem Randverlauf ausgebildet ist/sind und wobei die Soll-Biegestelle als Aussparung von Material und als Schwächung im Bereich der Breite des Halterungselements an einer Stelle ausgebildet ist, um leichter beim Einbringen in den Kapselsack nachzugeben und in der Lagerungsebene abzuknicken, **dadurch gekennzeichnet, dass** die Breite der Soll-Biegesetelle (5, 15, 16) in der Lagerungsebene (E) geringer ist als die Dicke in Richtung der optischen Achse (OA), wobei alle Halterungselemente (4, 14) als C-Haptiken ausgebildet sind und sich auf beiden Seiten der Soll-Biegestelle (5, 15, 16) ein Bereich anschließt, dessen Breite in der Lagerungsebene (E) mindestens so groß wie, insbesondere größer ist als die Dicke des entsprechenden Halterungselements (4, 14) in Richtung der optischen Achse (OA).

2. Intraokularlinse (1, 11) nach Anspruch 1, **dadurch gekennzeichnet, dass**:
• die Haptikvorrichtung (3, 13) zwei oder mehr Halterungselemente umfasst, und/oder
• die Halterungselemente (4, 14) in der gleichen Richtung gekrümmt angeordnet sind.

3. Intraokularlinse (1, 11) nach einem der vorgenannten Ansprüche, **dadurch gekennzeichnet, dass** die Halterungselemente (4, 14) um den Linsenkörper (2, 12) herum:
• bezüglich einer Linie in der Lagerungsebene (E) senkrecht zur optischen Achse (OA) spiegelsymmetrisch angeordnet sind, und/oder
• in der Lagerungsebene (E) in jeweils gleichen Winkelabständen bezüglich der Mitte des Linsenkörpers (2, 12) angeordnet sind.

4. Intraokularlinse (1, 11) nach einem der vorgenannten Ansprüche, **dadurch gekennzeichnet, dass** die Aussparung der wenigstens einen Soll-Biegestelle (5, 15, 16) offen ausgebildet ist.

5. Intraokularlinse (1, 11) nach einem der vorgenannten Ansprüche, **dadurch gekennzeichnet, dass** die Aussparung der wenigstens einen Soll-Biegestelle (5, 15, 16) auf der Innenseite des jeweiligen bogenförmigen Halterungselements (4, 14) angeordnet ist.

6. Intraokularlinse (1, 11) nach einem der vorgenannten Ansprüche, **dadurch gekennzeichnet, dass**
• der Linsenkörper (2, 12) und/oder die Hülle des Linsenkörpers sowie
die Haptikvorrichtung (3, 13) einteilig und/oder aus dem gleichen Material gefertigt sind.

## Claims

1. Intraocular lens (1, 11) as an implant for replacing a natural lens in the capsular bag of an eye, comprising:
• a lens body (2, 12) for forming the central optical lens, which has an optical axis (OA),
• a haptic device (3, 13) attached to the edge of the lens body (2, 12), for fixing the intraocular lens (1, 11) in the capsular bag and/or for stretching the capsular bag and/or for centring the lens body (2, 12) in the capsular bag,
• wherein the haptic device (3, 13) lies in a bearing plane (E) which is perpendicular to the optical axis (OA) of the lens body (2, 12),
• wherein the haptic device (3, 13) comprises at least one at least partially arcuate holding element (4, 14),
• wherein the at least one holding element (4, 14) has at least one predetermined bending point (5, 15, 16), wherein the predetermined bending point or several predetermined bending points (15, 16) on at least one of the holding elements (14) is/are designed as a recess with a smooth edge profile, and wherein the predetermined bending point is designed as a material cutout and as a weakening at one location in the region of the width of the holding element, in order to more easily yield during insertion into the capsular bag and to bend in the bearing plane, **characterized in that** the width of the predetermined bending point (5, 15, 16) in the bearing plane (E) is less than the thickness in the direction of the optical axis (OA), wherein all of the holding elements (4, 14) are designed as C-haptics and both sides of the predetermined bending point (5, 15, 16) are adjoined by a region whose width in the bearing plane (E) is at least as great as, in particular greater than, the thickness of the corresponding holding element (4, 14) in the direction of the optical axis (OA).

2. Intraocular lens (1, 11) according to Claim 1, **characterized in that**:
• the haptic device (3, 13) comprises two or more holding elements, and/or
• the holding elements (4, 14) are arranged curved in the same direction.

3. Intraocular lens (1, 11) according to either of the preceding claims, **characterized in that** the holding elements (4, 14) around the lens body (2, 12):
• are arranged mirror-symmetrically with respect to a line in the bearing plane (E) perpendicular to the optical axis (OA), and/or
• are arranged, in the bearing plane (E), at the same angular intervals with respect to the centre of the lens body (2, 12).

4. Intraocular lens (1, 11) according to any one of the preceding claims, **characterized in that** the recess of the at least one predetermined bending point (5, 15, 16) is designed open.

5. Intraocular lens (1, 11) according to any one of the preceding claims, **characterized in that** the recess of the at least one predetermined bending point (5, 15, 16) is arranged on the inner side of the respective arcuate holding element (4, 14).

6. Intraocular lens (1, 11) according to any one of the preceding claims, **characterized in that**
• the lens body (2, 12) and/or the envelope of the lens body and also
the haptic device (3, 13) are produced in one piece and/or from the same material.

## Revendications

1. Lentille intraoculaire (1, 11) en tant qu'implant pour le remplacement d'une lentille naturelle dans le sac capsulaire d'un œil, comprenant :
• un corps (2, 12) de lentille, destiné à former la lentille optique centrale, qui présente un axe optique (OA),
• un dispositif haptique (3, 13) disposé au niveau du bord du corps (2, 12) de lentille et destiné à fixer la lentille intraoculaire (1, 11) dans le sac capsulaire et/ou à étirer le sac capsulaire et/ou à centrer le corps (2, 12) de lentille dans le sac capsulaire,
• le dispositif haptique (3, 13) se situant dans un plan de logement (E) qui est perpendiculaire à l'axe optique (OA) du corps de lentille (2, 12),
• le dispositif haptique (3, 13) comprenant au moins un élément de fixation (4, 14) en forme d'arc par sections,
• ledit au moins un élément de fixation (4, 14) présentant au moins un site de flexion de consigne (5, 15, 16), le site de flexion de consigne ou plusieurs sites de flexion de consigne (15, 16) étant réalisé(s) au niveau d'au moins l'un des éléments de fixation (14) en tant qu'évidement avec un tracé de bord lisse et le site de flexion de consigne étant réalisé en tant qu'évidement de matériau et en tant qu'affaiblissement dans la zone de la largeur de l'élément de fixation au niveau d'un site afin de fléchir plus facilement lors de l'introduction dans le sac capsulaire et de s'infléchir dans le plan de logement, **caractérisée en ce que** la largeur du site de flexion de consigne (5, 15, 16) dans le plan de logement (E) est plus petite que l'épaisseur dans la direction de l'axe optique (OA), tous les éléments de fixation (4, 14) étant réalisés en tant qu'haptiques en C et une zone se raccordant sur les deux côtés du site de flexion de consigne (5, 15, 16) dont la largeur dans le plan de logement (E) est au moins aussi grande, en particulier plus grande, que l'épaisseur de l'élément de fixation (4, 14) correspondant dans la direction de l'axe optique (OA).

2. Lentille intraoculaire (1, 11) selon la revendication 1, **caractérisée en ce que**
• le dispositif haptique (3, 13) comprend deux éléments de fixation ou plus et/ou
• les éléments de fixation (4, 14) sont agencés de manière incurvée dans la même direction.

3. Lentille intraoculaire (1, 11) selon l'une des revendications précédentes, **caractérisée en ce que** les éléments de fixation (4, 14) sont agencés autour du corps (2, 12) de lentille :
• de manière spéculairement symétrique par rapport à une ligne dans le plan de logement (E) perpendiculaire l'axe optique (OA) et/ou
• dans le plan de logement (E) à des distances angulaires à chaque fois identiques par rapport au centre du corps (2, 12) de lentille.

4. Lentille intraoculaire (1, 11) selon l'une des revendications précédentes, **caractérisée en ce que** l'évidement dudit au moins un site de flexion de consigne (5, 15, 16) est réalisé de manière ouverte.

5. Lentille intraoculaire (1, 11) selon l'une des revendications précédentes, **caractérisée en ce que** l'évidement dudit au moins un site de flexion de consigne (5, 15, 16) est agencé sur la face interne dudit élément de fixation (4, 14) en forme d'arc respectif.

6. Lentille intraoculaire (1, 11) selon l'une des revendications précédentes, **caractérisée en ce que**
• le corps (2, 12) de lentille et/ou l'enveloppe du corps de lentille ainsi que
le dispositif optique (3, 13) sont fabriqués d'une seule pièce et/ou à partir du même matériau.
